# EUROPEAN PATENT SPECIFICATION

(11) **EP 1 877 112 B1**
(45) Date of publication and mention of the grant of the patent: **28.12.2011**
(21) Application number: 06752339.9
(22) Date of filing: 04.05.2006
(51) Int. Cl.: A61L 31/02, A61L 31/18, A61F 2/06

(54) **MEDICAL DEVICES AND METHODS OF MAKING THE SAME**
MEDIZINISCHE VORRICHTUNGEN UND VERFAHREN ZU IHRER HERSTELLUNG
DISPOSITIF MEDICAUX ET PROCEDES POUR LES PRODUIRE

(30) Priority: 05.05.2005 US 122819
(43) Date of publication of application: 16.01.2008
(73) Proprietor: Boston Scientific Limited, Hastings Christ Church (BB)
(72) Inventor: LENZ, Jason, Maplewood, Minnesota 55109 (US)
(74) Representative: Peterreins, Frank
(86) International application number: PCT/US2006/017475
(87) International publication number: WO 2006/121890

(56) References cited:
- WO-A-2005/058537
- WO-A1-2006/042230
- US-A- 6 099 561
- US-A1- 2002 010 505
- US-A1- 2004 000 046
- US-A1- 2004 106 982
- US-B1- 6 767 360

## Description

### TECHNICAL FIELD

The invention relates to medical devices, such as stents, and methods of making the devices.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

Document US 2004/106982 shows a metallic three layer stent; documents US 2004/000046, US 2002/010505, US 6767360, US 6099561 disclose other endoprosthesys structures comprising multiple layers for enhencing visibility (MRI) and/or biocompatibility.

### SUMMARY

The invention relates to medical devices, such as stents, and methods of making the medical devices, as disclosed in the appended claims.

In one aspect, the invention features an endoprosthesis that includes at least three layers. Each of the layers includes approximately one grain or more across a thickness of the layer, and each of the layers has substantially the same composition.

In another aspect, the invention features a method of making an endoprosthesis. The method includes forming a tubular member defining a lumen into the endoprosthesis. The tubular member includes at least three layers having substantially the same composition, and each of the layers includes approximately one grain or more across a thickness of the layer.

In a further aspect, the invention features a method of making an endoprosthesis, which includes arranging a plurality of tubes substantially concentrically about one another to form a tubular member that includes multiple layers. The tubular member comprises at least three grains across a thickness of the tubular member. The method also includes decreasing a thickness of at least one of the layers.

Embodiments can include one or more of the following features.

In some embodiments, at least some of the grains have an ASTM E112 grain value of at least about six (e.g., about six to about 12).

In certain embodiments, at least one of the layers has a thickness of about 0.08 mm or less (e.g., 0.01 mm to 0.08 mm).

At least one of the layers includes stainless steel, an alloy comprising platinum and stainless steel, niobium, tantalum, titanium, iridium, cobalt, and/or chromium.

In certain embodiments, the endoprosthesis further includes a first material disposed between two of the layers, the first material having a different composition than a composition of the two layers.

In some embodiments, the first material is disposed between each of the layers.

In certain embodiments, the first material comprises a stainless steel alloy.

In some embodiments, the endoprosthesis further includes multiple bands and connectors.

In certain embodiments, at least one of the bands includes multiple layers.

In some embodiments, at least one of the bands has a thickness of 0.01 mm to 0.08 mm.

In some embodiments, the method further includes decreasing a thickness of at least one of the layers.

In certain embodiments, after decreasing the thickness, the thickness of the at least one of the layers is 0.08 mm or less (e.g., 0.01 mm to 0.08 mm).

In some embodiments, the method includes concentrically arranging a plurality of tubes to form the tubular member.

In certain embodiments, the method further includes work hardening the tubular member such that at least some of the grains are reduced in size.

In some embodiments, the method further includes disposing a first material between at least some of the layers, the first material having a different composition than a composition of one of the layers.

In certain embodiments, the method includes disposing the first material between each of the layers.

In some embodiments, the method further includes removing portions of the tubular member to form multiple bands and connectors.

In certain embodiments, at least one of the connectors has a width of 0.03 mm to 0.3 mm.

In some embodiments, the tubular member comprises at least three layers.

In certain embodiments, each of the layers comprises approximately one grain or more across a thickness of the layer.

In some embodiments, at least one of the layers comprises approximately one grain across the thickness of the layer.

Embodiments may include one or more of the following advantages.

In some embodiments, medical devices, such as endoprostheses, can be made with an arrangement of grains such that the endoprostheses can be thin and strong. The endoprostheses, therefore, can assume a small profile when compacted, and can be delivered through and implanted within bodily vessels having relatively small diameters.

In certain embodiments, the methods of making the devices allow reliable formation of devices having multiple grains across the thickness of the device.

Other aspects, features, and advantages of the invention will be apparent from the description of the preferred embodiments thereof and from the claims.

### DESCRIPTION OF DRAWINGS

Fig. 1A is a perspective view of an embodiment of a stent.
Fig. 1B is an enlarged view of area 1B in Fig. 1A.
Fig. 2 is a partial, cross-sectional view of the stent of Fig. 1 taken along lines 2-2.
Fig. 3 is an enlarged view of area 3 in Fig. 2.
Fig. 4 is a flowchart illustrating a method of making the stent of Fig. 1.

### DETAILED DESCRIPTION

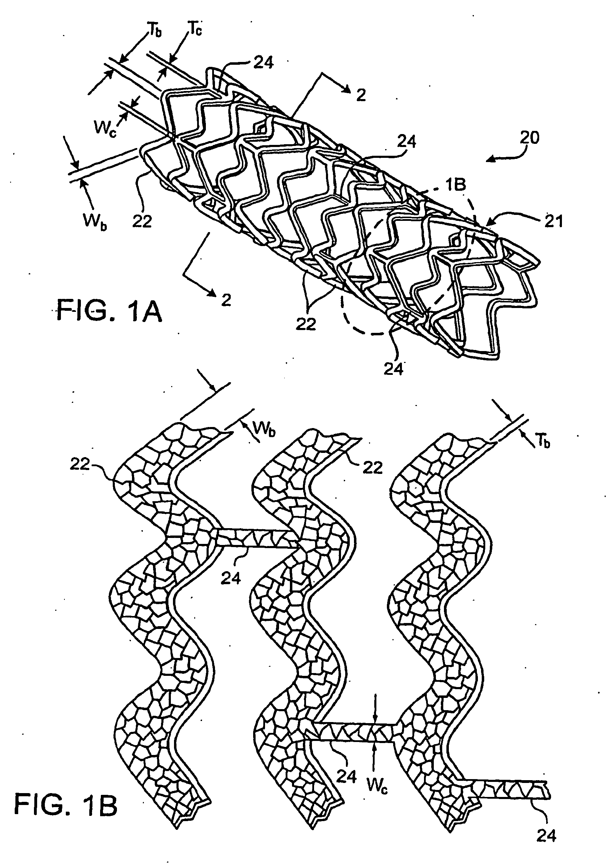

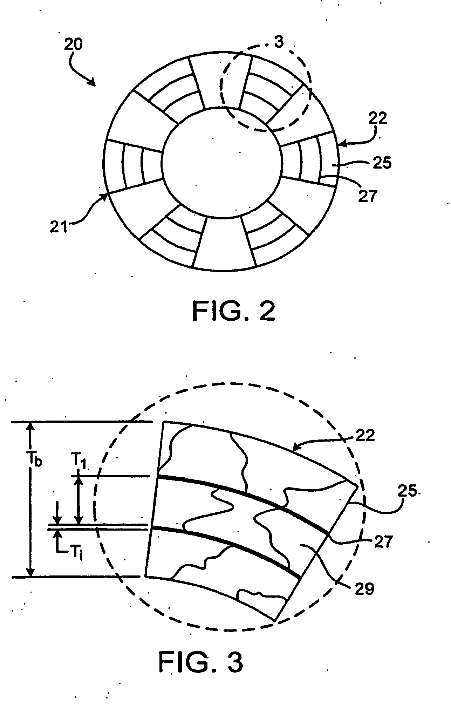

Referring to Figs. 1A and 1B, a stent 20 has the form of a tubular member 21 defined by a plurality of bands 22 and a plurality of connectors 24 that extend between and connect adjacent bands. Referring to Figs. 2 and 3, tubular member 21 is a multi-layered structure having multiple layers 25 and multiple interfacial layers 27 (as shown, three layers 25 alternating with two interfacial layers 27). Each of layers 25 includes one or more grains 29 across the thickness of the layer. Consequently, tubular member 21 includes multiple grains (as shown, three or more) across its thickness. Without wishing to be bound by theory, it is believed that this grain microstructure can enhance the mechanical properties of stent 20.

During use, a stent can experience relatively high levels of stress and fatigue. For example, the stent can be bent as it tracks through a tortuous vessel during delivery, as it is expanded, and/or when it is placed in a curved vessel. After implantation, the stent can also experience stress from movement caused by a beating heart or by the subject's breathing. The stress can strain the bands and connectors, and can even fracture the bands and/or connectors, for example. A fractured band or connector can provide surfaces that disrupt blood flow and/or provide sites on which blood can aggregate and undesirably lead to blood clotting or thrombosis in the vessel. By making the stent with a selected grain microstructure (e.g., three or more grains across the thickness of the stent), the fatigue resistance of the stent is enhanced. Consequently, the bands and connectors can better tolerate the stress that can lead to fracture, while still being easily deformable.

Furthermore, in some methods of making a stent, the stent is subjected to one or more heat treatment steps that can lead to an unwanted microstructure. For example, while a heat treatment may lead to a desirable microstructure (such as equiaxed grains), the heat treatment can also cause grain growth and a reduction of the number of grains across a thickness of the stent. As a result, the stent may have an undesired fatigue resistance. However, as described below, by making stent 20 from multiple layers 25 and controlling the interaction between the layers during fabrication (e.g., by using interfacial layers 27 and/or by controlling heat treatment), the grain structure can be controlled, and thus the fatigue resistance of the stent can be improved.

Layers 25 can include (e.g., can be manufactured from) one or more biocompatible materials with mechanical properties so that stent 20 can be compacted, and subsequently expanded to support a vessel. In some embodiments, stent 20 can have an ultimate tensile strength (UTS) of 20-160 ksi, greater than about 15% elongation to failure, and a modulus of elasticity of 10-60 msi. When stent 20 is expanded, the material can be stretched to strains on the order of 30 percent. Examples of materials include stainless steel (e.g., 316L and 304L stainless steel, and an alloy including stainless steel and 5-60% by weight of one or more radiopaque elements (e.g., Pt, Ir, Au, W) (PERSS®) as described in US-2003-0018380-A1, US-2002-0144757-A1, and US-2003-0077200-A1), Nitinol (a nickel-titanium alloy), Elgiloy, L605 alloys, MP35N, titanium, titanium alloys (e.g., Ti-6A1-4V, Ti-50Ta, Ti-10Ir), platinum, platinum alloys, niobium, niobium alloys (e.g., Nb-IZr) Co-28Cr-6Mo, tantalum, and tantalum alloys. Other examples of materials are described in commonly assigned U.S.S.N. 10/672,891, filed September 26, 2993, and entitled "Medical Devices and Methods of Making Same"; and U.S.S.N. 11/035,316, filed January 3, 2005, and entitled "Medical Devices and Methods of Making Same". Other materials include elastic biocompatible metal such as a superelastic or pseudo-elastic metal alloy, as described, for example, in Schetsky, L. McDonald, "Shape Memory Alloys", Encyclopedia of Chemical Technology (3rd ed.), John Wiley & Sons, 1982, vol. 20. pp. 726-736; and commonly assigned U.S.S.N. 10/346,487, filed January 17, 2003.

The material(s) can include one or more radiopaque materials to provide radiopacity. Examples of radiopaque materials include metallic elements having atomic numbers greater than 26 (e.g., greater than 43). In some embodiments, the radiopaque materials have a density greater than 9.9 g/cc. In certain embodiments, the radiopaque material is relatively absorptive of X-rays, e.g., having a linear attenuation coefficient of at least 25 cm⁻¹ (e.g., at least 50 cm⁻¹) at 100 keV. Examples of radiopaque materials include tantalum, platinum, iridium, palladium, hafnium, tungsten, gold, ruthenium, and rhenium. The radiopaque material can include an alloy, such as a binary, a ternary or more complex alloy, containing one or more elements listed above with one or more other elements such as iron, nickel, cobalt, or titanium. Examples of alloys including one or more radiopaque materials (e.g., PERSS®) are described in U.S. Application Publication US-2003-0018380-A1; US-2002-0144757-A1; and US-2003-0077200-A1.

In some embodiments, stent 20 includes one or more materials that enhance visibility by magnetic resonance imaging (MRI). Examples of MRI materials include non-ferrous metal-alloys containing paramagnetic elements (e.g., dysprosium or gadolinium) such as terbium-dysprosium, dysprosium, and gadolinium; non-ferrous metallic bands coated with an oxide or a carbide layer of dysprosium or gadolinium (e.g., Dy₂O₃ or Gd₂O₃); non-ferrous metals (e.g., copper, silver, platinum, or gold) coated with a layer of superparamagnetic material, such as nanocrystalline Fe₃O₄, CoFe₂O₄, MnFe₂O₄, or MgFe₂O₄; and nanocrystalline particles of the transition metal oxides (e.g., oxides of Fe, Co, Ni). Alternatively or additionally, stent 20 can include one or more materials having low magnetic susceptibility to reduce magnetic susceptibility artifacts, which during imaging can interfere with imaging of tissue adjacent to and/or surrounding the stent, for example. Low magnetic susceptibility materials include tantalum, platinum, titanium, niobium, copper, and alloys containing these elements. The MRI visible materials can be incorporated into the structural material, can serve as the structural material, and/or be included as a layer of stent 20.

Layers 25 can each have the same composition, can each have a different compositions, or can have various combinations of compositions.

The thickness of layer 25 (T₁) can be a function of the number of layers 25 in tubular member 21, the composition of layers 25, the targeted mechanical properties, and/or the type of stent. In some embodiments, thickness (T₁) of layer 25 can range from 0.01 mm to 0.08 mm. For example, thickness T₁ can be less than or equal to 0.08 mm (e.g., less than or equal to 0.06 mm, less than or equal to 0.04 mm, less than or equal to 0.02 mm). Each of layers 25 of a stent can have the same thickness, can have different thicknesses, or can have various combinations of thickness.

Interfacial layers 27 can include one or more biocompatible materials capable of interrupting grain boundaries between adjacent layers 25. Interfacial layers 27, for example, can include any of various materials that are capable of forming a strong bond with adjacent layers, while having a different grain size or structure than the adjacent layers. In some embodiments, the material(s) of interfacial layers 27 have different grain growth properties than the adjacent layers. Interfacial layers 27 can include any of the various materials described above with respect to layers 25.

Similar to layers 25, the thickness of interfacial layers 27 (T₁) can be a function of the number of layers 27 in tubular member 21, the composition of layers 27, the targeted mechanical properties, and/or the type of stent. In some embodiments, thickness (Tᵢ) of layer 27 can range from 0.01 mm to 0.08 mm. For example, thickness Tᵢ can be less than or equal to 0.08 mm (e.g., less than or equal to 0.06 mm, less than or equal to 0.04 mm, less than or equal to 0.02 mm). The thickness of layers 27 of a stent can be the same or different.

Together, layers 25 and interfacial layers 27 form the thickness T_{b} of bands 22 of stent 20. In some embodiments, thickness T_{b} can range from 0.05 mm to 0.2 mm. For example, thickness T_{b} of bands 22 can be less than or equal to 0.2 mm (e.g., less than or equal to 0.1 mm, less than or equal to 0.08 mm, less than or equal to about 0.06 mm).

Along thickness T_{b}, layers 25 and interfacial layers 27 form three or more layers. For example, stent 20 can include four or more layers, five or more layers, six or more layers, or seven or more layers. In some embodiments, stent 20 includes no interfacial layers 27 between adjacent layers 25. In other embodiments, stent 20 includes multiple interfacial layers 27 between adjacent layers 25.

Each layer 25 can include one or more grains 29 across its thickness Tᵢ. As a result, the number of grains 29 across stent 20 can be a function of the number of layers 25. As the number of layers increases, the number of grains across thickness T_{b} of stent 20 also increases. Increasing the number of grains 29 (or layers 25) across thickness T_{b} of stent 20 can increase the fatigue strength of the stent and provide a favorable distribution of the stresses along the stent.

In some embodiments, one or more of the layers has grains 29 with an average ASTM E112 value of from about six to about 12 (the ASTM E112 value being inversely proportional to the average grain diameter). For example, the ASTM E112 value can be greater than or equal to six, seven, eight, nine, 10, 11, or 12. In certain embodiments, the average grain diameter can range from five microns to 45 microns. For example, the average grain diameter can be less than or equal to 45 microns, 40 microns, 35 microns, 30 microns, 25 microns, 20 microns, 15 microns, or ten microns; and/or greater than or equal to five microns, ten microns, 15 microns, 20 microns, 25 microns, 30 microns, 35 microns, or 40 microns. In embodiments in which stent 20 includes one or more refractory metals, such as niobium, tantalum, or tungsten, the grain size can be fine (e.g., 25 microns or less, 20 microns or less, 15 microns or less, 10 microns or less) to reduce brittleness.

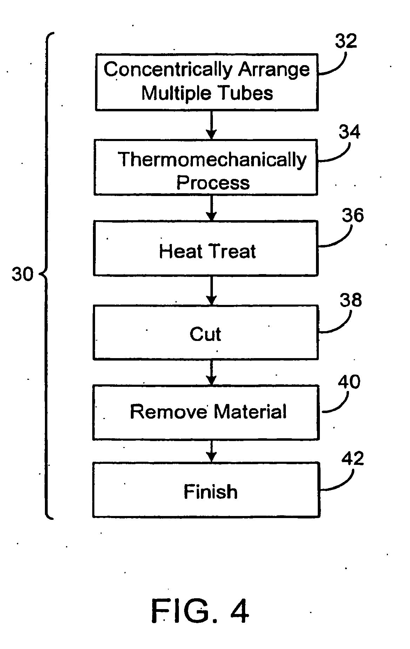

Fig. 4 shows a method 30 of making stent 20. As shown, method 30 includes concentrically arranging multiple tubes (step 32) one another. The multiple tubes are then drawn down (step 34) to decrease the thickness of the multiple layers. As a result, a multi-layered tubular member is formed. The multi-layered tubular member is heat-treated (step 36) to create a bond (e.g., a diffusion bond) between the multiple layers and to alter the grain structure of the materials from which the layers are formed. The multi-layered tubular member is subsequently cut to form bands 22 and connectors 24 (step 38) to produce an unfinished stent. Areas of the unfinished stent affected by the cutting are subsequently removed (step 40). Finally, the unfinished stent is finished by electropolishing, for example, to form stent 20 (step 42).

Still referring to Fig. 4, the first step of method 30 includes arranging multiple tubes concentrically about one another (step 32) in order to form the multi-layered tubular member from which stent 20 is made. More specifically, multiple tubes having varying diameters are fitted over one another. The tubes, for example, can be configured in a "slip-fit" arrangement. The tube having the smallest diameter is ultimately positioned at the innermost portion of the resulting tubular member, while the tube having the largest diameter is ultimately positioned at the outermost portion of the tubular member. The (inner or outer) diameters of the tubes can range from 0.5 mm to five mm. The multiple tubes, after processing, become layers 25 of stent 20 (shown in Figs. 2 and 3).

In some embodiments, one or more interfacial layers 27 (shown in Figs. 2 and 3) are disposed between adjacent tubes of layers 25. Interfacial layer(s) 27 can help to prevent the grains of adjacent tubes from merging during the heat-treatment. Interfacial layer(s) 27 can, for example; be one or more additional tubes concentrically arranged within the multi-layered tubular member. In other embodiments, interfacial layer(s) 27 can be deposited on the tubes using any of various other techniques, such as chemical vapor deposition, physical vapor deposition, and/or sputtering. For example, prior to concentrically arranging the tubes, interfacial layer(s) 27 can be applied to the tubes using one or more of the above-noted techniques. In some embodiments, interfacial layer(s) 27 is disposed between each of the adjacent tubes. In certain embodiments, multiple interfacial layers 27 (e.g., multiple tubes corresponding to interfacial layers 27) are arranged between adjacent tubes of layers 25. The interface material (e.g., the material from which interfacial layer(s) 27 is formed) can have a composition different than that of the tubes of layers 25.

After concentrically arranging the multiple tubes, as described above, the resulting tubular member is processed (e.g., thermomechanically processed) to decrease the thickness of the tubes (step 34). For example, the tubular member can be drawn through a series of dies with progressively smaller circular openings to plastically deform the member to a targeted size and shape. By drawing the tubular member through the series of dies, the thickness of the tubes can be decreased to form multiple layers of a desired thickness. In some embodiments, the plastic deformation strain hardens the member (and increases its yield strength) and elongates the grains along the longitudinal axis of the member. The drawing process can also create a mechanical bond between the adjacent tubes. For example, the relatively high pressure and high temperature experienced during the drawing process can cause the adjacent tubes to bond with one another.

In certain embodiments, the steps of arranging the multiple tubes and processing the tubes can be performed in an alternating fashion. For example, two tubes can be concentrically arranged about one another and then processed. Subsequently, a third tube can be arranged concentrically about the processed tubes to form a three-layer tubular member, which can then be processed. This alternating procedure can be repeated until a tubular member having a desired number of layers is achieved.

After being processed, the tubular member can be heat-treated to change its microstructure (step 36). For example, the tubular member can be annealed above the recrystallization temperature and/or hot isostatically pressed to transform the elongated grain structure into an initial grain structure, e.g., one including equiaxed grains. Small or fine grains can be formed by heating the member close to the recrystallization temperature for a relatively short time. Large or coarse grains can be formed by heating the member at higher temperatures and/or for longer times to promote grain growth. During the heat-treatment, grains have the tendency to grow and to merge with one another, but by using interfacial layer(s) 27, the grains of adjacent tubes can be prevented from merging with one another. Grains of the respective layers (e.g., interfacial layer(s) 27 and tubes) can, for example, grow substantially independently of one another. As a result, multiple layers and multiple grains can be maintained throughout the heat-treatment, and the fatigue strength of the tubular member can be enhanced.

The thermomechanical processing step (step 34) and the heat-treating step (step 36) can be repeated until the tubular member has a desired thickness and grain structure. In some embodiments, these steps are performed two (e.g., three, four, five, ten) times or more.

In certain embodiments, the tubular member can be heat-treated prior to completely forming the multi-layered tubular member. For example, in embodiments in which the multiple tubes are concentrically arranged and processed in alternating steps, the partially formed tubular member can be heat-treated between one or more of the tube-arranging steps and the processing steps. Alternatively or additionally, the tubes can be heat-treated prior to being concentrically arranged to form the multi-layered tubular member.

In some embodiments, one or more portions of the tubular member can be selectively masked prior to the heat-treatment in order to produce varying grain structures across the stent. As a result, the stent can include regions of varying physical properties, such as strength, rigidity, and ductility. For example, selected portions of the tubular member can be coated with a polished and reflective coating (e.g., on the connectors) and/or a blackened coating (e.g., on the bands). The polished and reflective coating (such as gold, platinum, and/or silver) can reduce the amount of heat transferred to the tubular member. The blackened coating (such as graphite) can increase the amount of heat transferred to the tubular member.

Next, bands 22 and connectors 24 of multi-layered stent 20 are formed, as shown, by cutting the tube (step 38). For example, selected portions of the tube can be removed to form bands 22 and connectors 24 by laser cutting, as described in U.S. Patent No. 5,780,807. In certain embodiments, during laser cutting, a liquid carrier, such as a solvent or an oil, is flowed through the lumen of the tube. The carrier can prevent dross formed on one portion of the tube from re-depositing on another portion, and/or reduce formation of recast material on the tube. Alternatively or additionally, other methods of removing portions of the tube can be used, such as mechanical machining (e.g., micro-machining), electrical discharge machining (EDM), and photoetching (e.g., acid photoetching).

In some embodiments, after bands 22 and connectors 24 are formed, areas of the multi-layered tube affected by the cutting operation above can be removed (step 40). For example, laser machining of bands 22 and connectors 24 can leave a surface layer of melted and resolidified material and/or oxidized metal that can adversely affect the mechanical properties and performance of multi-layered stent 20. The affected areas can be removed mechanically (such as by grit blasting or honing) and/or chemically (such as by etching or electropolishing). In some embodiments, the tubular member can be near-net size after step 36 is performed. "Near-net size" means that the tube has a relatively thin envelope of material that is removed to provide a finished stent. In some embodiments, the tube is formed less than 25% oversized, e.g., less than 15%, 10%, or 5% oversized.

The unfinished stent is then finished (step 42) to form stent 20. The unfinished stent can be finished, for example, by electropolishing to a smooth finish. Since the unfinished stent can be formed to near-net size, relatively little of the unfinished stent needs to be removed to finish the stent. As a result, further processing (which can damage the stent) and costly materials can be reduced. In some embodiments, 0.0025 mm of the stent material can be removed by chemical milling and/or electropolishing to yield a stent.

Stent 20 can be of any desired shape and size (e.g., coronary stents, aortic stents, peripheral vascular stents, gastrointestinal stents, urology stents, and neurology stents). Depending on the application, stent 20 can have a diameter of one mm to 46 mm. In certain embodiments, a coronary stent can have an expanded diameter of from two mm to six mm. In some embodiments, a peripheral stent can have an expanded diameter of from five mm to 24 mm. In certain embodiments, a gastrointestinal and/or urology stent can have an expanded diameter of from six mm to 30 mm. In some embodiments, a neurology stent can have an expanded diameter of from one mm to 12 mm. An abdominal aortic aneurysm (AAA) stent and a thoracic aortic aneurysm (TAA) stent can have a diameter from 20 mm to 46 mm. Stent 20 can be balloon-expandable, self-expandable, or a combination of both (e.g., U.S. Patent No. 5,366,504).

In use, stent 20 can be used, e.g., delivered and expanded, using a catheter delivery system. Catheter systems are described in, for example, Wang U.S. 5,195,969, Hamlin U.S. 5,270,086, and Raeder-Devens, U.S. 6,726,712. Stents and stent delivery are also exemplified by the Radius® or Symbiot® systems, available from Boston Scientific Scimed, Maple Grove, MN.

While a number of embodiments have been described above, the invention is not so limited.

As an example, connectors 24 can have different dimensions than bands 22 and have a multi-layered construction. For example, the width of connectors 24 (W_{c}, shown in Fig. 1) can be less than the widths of bands 22 (W_{b}) to allow the connectors to flex and to conform to a vessel. As used herein, a connector 24 refers to a portion of a stent that extends from a band of the stent, for example, from a first band to an adjacent second band along the length of the stent. The connector can include one strut (as shown in Fig. 1) or a plurality of struts. The connector can extend linearly (e.g., parallel to the longitudinal axis of the stent) or nonlinearly, for example, in an undulating patter or zigzag pattern. As used herein, a band 22 refers to a portion of a stent that extends circumferentially about the stent. The band can extend completely about the circumference of a stent, for example, such that the ends of the band are joined, or the band can extend partially about the circumference. The band can extend substantially linearly or nonlinearly, for example, in an undulating pattern or a zigzag pattern as shown in Figs. 1A and 1B. In some embodiments, bands 22 are connected together by integrally formed connectors that extend between and transversely to the bands. Some examples of dimensions for bands 22 are disclosed in commonly assigned U.S.S.N. 10/961,289, filed October 8, 2004, and entitled "Medical Devices and Methods of Making the Same".

In some embodiments the width W_{c} of connector 24 ranges from 0.030 mm to 0.3 mm. Particular widths of connector 24 can be a function of the material(s) in stent 20, the type of stent and/or the desired performance. For example, connector width W_{c} of a stent including 316L stainless steel can range from 0.05 mm to 0.2 mm; connector width W_{c} of a stent including a PERSS® alloy can range from 0.03 mm to 0.18 mm; connector width W_{c} of a stent including an alloy having chromium and cobalt can range from 0.02 mm to 0.16 mm; connector width W_{c} of a stent including a refractory metal can range from 0.08 mm to 0.30 mm; and connector width W_{c} of a stent including an alloy having titanium can range from 0.03 mm to 0.15 mm.

In some embodiments, connector 24 includes one or more grains across width W_{c}. For example, connector 24 can have at least three grains (e.g., at least four grains, at least five grains, or at least six grains) across width W_{c}. In some embodiments, connector 24 has an average ASTM E112 grain size of about six or smaller. The average grain diameter, for example, can range from five microns to 45 microns. For example, the average grain diameter can be equal to or less than 45 microns, 40 microns, 35 microns, 30 microns, 25 microns, 20 microns, 15 microns, or ten microns; and/or greater than or equal to five microns, ten microns, 15 microns, 20 microns, 25 microns, 30 microns, 35 microns, or 40 microns. In embodiments in which connectors 24 include one or more refractory metals, such as niobium, tantalum, or tungsten, the grain size can be fine (e.g., less than or equal to 25 microns, 20 microns, 15 microns, or ten microns) to reduce brittleness.

In certain embodiments, connector 24 can include multiple grains (e.g., three or more) across a thickness T_{c} (shown in Fig. 1) of connector 24 as described above for tubular member 21.

In some embodiments, stent 20 includes at least one layer of a softer material and at least one layer of a harder material. For example, a layer formed of a soft malleable material can be positioned adjacent a layer formed of a higher strength material. Consequently, recoil of the stent, which can cause problems among stents formed of high strength materials, can be reduced.

Stent 20 can also be a part of a covered stent or a stent-graft. In some embodiments, stent 20 can include and/or be attached to a biocompatible, non-porous or semi-porous polymer matrix made of polytetrafluoroethylene (PTFE), expanded PTFE, polyethylene, urethane, or polypropylene.

Stent 20 can include a releasable therapeutic agent, drug, or a pharmaceutically active compound, such as described in U.S. Patent No. 5,674,242, U.S.S.N. 09/895,415, filed July 2,2001, and U.S.S.N. 10/232,265, filed August 30, 2002. The therapeutic agents, drugs, or pharmaceutically active compounds can include, for example, anti-thrombogenic agents, antioxidants, anti-inflammatory agents, anesthetic agents, anti-coagulants, and antibiotics.

In other embodiments, the structures and methods described herein can be used to make other medical devices. For example, the structures and methods described herein can be used to make devices, such as hypotube catheter shafts and/or guide wires.

Other embodiments are within the claims.

## Claims

1. An endoprosthesis comprising at least three layers (25), each of the layers comprising one grain (29) or more across a thickness of the layer (25), **characterized in that** the layers have substantially the same composition, and **in that** an interfacial layer (27) is disposed between each of the layers (25) preventing grains of adjacent layers from merging with one another, wherein at least one of the layers includes stainless steel, an alloy comprising platinum and stainless steel, niobitun, tantalum, titanium, iridium, cobalt, and/or chromium.

2. The endoprosthesis of claim 1, wherein at least some of the grains (29) have an ASTM El 12 grain value of at least six.

3. The endoprosthesis of claim 1, wherein at least some of the grains (29) have an ASTM El 12 grain value of six to 12.

4. The endoprosthesis of claim 1, wherein at least one of the layers (25) has a thickness of 0.08 mm or less.

5. The endoprosthesis of claim 4, wherein the thickness of the at least one of the layers (25) is 0.01 mm to 0.08 mm.

6. The endoprosthesis of claim 1, wherein interfacial layer (27)comprises a first material having a different composition than a composition of the two layers.

7. The endoprosthesis of claim 6, wherein said first material includes one or more biocompatible materials capable of interrupting grain boundaries between adjacent layers (25).

8. The endoprosthesis of claim 6, wherein the first material comprises a stainless steel alloy.

9. The endoprosthesis of claim 1, further comprising a plurality of bands (22) and connectors (24).

10. The endoprosthesis of claim 9, wherein at least one of the bands (22) comprises the plurality of layers (25).

11. The endoprosthesis of claim 9, wherein at least one of the bands (22) has a thickness of 0.01 mm to 0.08 mm.

12. A method of making an endoprosthesis, the method comprising:
forming a tubular member defining a lumen into the endoprosthesis, the tubular member comprising at least three layers (25), each of the layers comprising one grain (29) or more across a thickness of the layer (25), wherein at least one of the layers include stainless steel, an alloy comprising platinum and stainless steel, niobium, tantalum, titanium, iridium, cobalt, and/or chromium;
**characterized in that** the layers (25) have substantially the same composition, and **in that** the method comprises the further step of disposing an interfacial layer (27) between each of the layers (25) preventing grains of adjacent layers from merging with one another.

13. The method of claim 12, further comprising decreasing a thickness of at least one of the layers (25).

14. The method of claim 13, wherein after decreasing the thickness, the thickness of the at least one of the layers (25) is 0.08 mm or less.

15. The method of claim 13, wherein after decreasing the thickness, the thickness of the at least one of the layers (25) is 0.01 mm to 0.08 mm.

16. The method of claim 12, comprising concentrically arranging a plurality of tubes to form the tubular member.

17. The method of claim 12, wherein at least some of the grains have an ASTM El 12 grain value of six or larger.

18. The method of claim 17, wherein the at least some of the grains have an ASTM El 12 grain value of six to 12.

19. The method of claim 12 , further comprising work hardening the tubular member such that at least some of the grains are reduced in size.

20. The method of claim 12, wherein interfacial layer (27) comprises a first material having a different composition than a composition of one of the layers.

21. The method of claim 20, wherein said first material includes one or more biocompatible materials capable of interrupting grain boundaries between adjacent layers (25).

22. The method of claim 12, further comprising removing portions of the tubular member to form a plurality bands (22) and connectors (24).

23. The method of claim 22, wherein at least one of the bands (22) has a thickness of 0.01 mm to 0.08 mm.

24. The method of claim 22, wherein the at least one of the bands (22) comprises the plurality of layers (25).

25. The method of claim 22, wherein the at least one of the connectors (24) has a width of 0.03 mm to 0.3 mm.

## Patentansprüche

1. Eine Endoprothese umfassend zumindest drei Schichten (25), jede der Schichten umfassend ein Korn (29) oder mehr über die Dicke der Schicht (25), **dadurch gekennzeichnet, dass** die Schichten im Wesentlichen die gleiche Zusammensetzung haben und dass eine Grenzflächenschicht (27) zwischen jeder der Schichten (25) angeordnet ist, die verhindert dass sich Körner angrenzender Schichten mit einander verbinden, wobei zumindest eine der Schichten Edelstahl, eine Legierung umfassend Platin und Edelstahl, Niobium, Tantal, Titan, Iridium, Kobalt und/oder Chrom beinhaltet.

2. Die Endoprothese nach Anspruch 1, wobei zumindest einige der Körner (29) eine ASTM E1 12 Korngröße von zumindest sechs haben.

3. Die Endoprothese nach Anspruch 1, wobei zumindest einige der Körner (29) eine ASTM E1 12 Korngröße von sechs bis zwölf haben.

4. Die Endoprothese nach Anspruch 1, wobei zumindest eine der Schichten (25) eine Dicke von 0.08 mm oder weniger hat.

5. Die Endoprothese nach Anspruch 4, wobei die Dicke der zumindest einen Schicht (25) 0.01 mm bis 0.08 mm ist.

6. Die Endoprothese nach Anspruch 1, wobei die Grenzflächenschicht (27) ein erstes Material umfasst, das eine andere Zusammensetzung hat als eine Zusammensetzung der zwei Schichten.

7. Die Endoprothese nach Anpsruch 6, wobei das erste Material ein oder mehrere biokompatible Materialien beinhaltet, die geeignet sind Korngrenzen zwischen angrenzenden Schichten (25) zu unterbrechen.

8. Die Endoprothese nach Anspruch 6, wobei das erste Material eine Edelstahllegierung umfasst.

9. Die Endoprothese nach Anspruch 1, weiter umfassend eine Mehrzahl von Bändern (22) und Verbindern (24).

10. Die Endoprothese nach Anspruch 9, wobei zumindest eines der Bänder (22) eine Mehrzahl von Schichten (25) umfasst.

11. Die Endoprothese nach Anspruch 9, wobei zumindest eines der Bänder (22) eine Dicke von 0,01 mm bis 0,08 mm hat.

12. Ein Verfahren zum Herstellen einer Endoprothese, das Verfahren umfassend:
Formen eines röhrenförmigen Teils das ein Lumen in der Endoprothese definiert, wobei der röhrenförmige Teil zumindest drei Schichten (25) umfasst, jede der Schichten umfassend ein Korn (29) oder mehr über die Dicke der Schicht (25), wobei zumindest eine der Schichten Edelstahl, eine Legierung umfassend Platin und Edelstahl, Niobium, Tantal, Titan, Iridium, Kobalt und/oder Chrom beinhaltet;
**dadurch gekennzeichnet, dass** die Schichten (25) im Wesentlichen die gleiche Zusammensetzung haben und dass das Verfahren den weiteren Schritt des Anordnens einer Grenzflächenschicht (27) zwischen jeder der Schichten (25) umfasst, die verhindert, dass sich Körner angrenzender Schichten mit einander verbinden.

13. Das Verfahren nach Anspruch 12, weiter umfassend Vermindern der Dicke von zumindest einer der Schichten (25).

14. Das Verfahren nach Anspruch 13, wobei nach Vermindern der Dicke, die Dicke von zumindest einer der Schichten (25) 0,08 mm oder weniger ist.

15. Das Verfahren nach Anspruch 13, wobei nach Vermindern der Dicke, die Dicke von zumindest einer der Schichten (25) 0,01 mm bis 0,08 mm ist.

16. Das Verfahren nach Anspruch 12, umfassend konzentrisches Anordnen einer Mehrzahl von Röhren um ein röhrenförmiges Teil zu formen.

17. Das Verfahren nach Anspruch 12, wobei zumindest einige der Körner eine ASTM E1 12 Korngröße von sechs oder größer haben.

18. Das Verfahren nach Anspruch 17, wobei die zumindest einige Körner eine ASTM E1 12 Korngröße von sechs bis 12 haben.

19. Das Verfahren nach Anspruch 12, weiter umfassend Härten des röhrenförmigen Teils so dass zumindest einige der Körner in der Größe verringert werden.

20. Das Verfahren nach Anspruch 12, wobei die Grenzflächenschicht (27) ein erstes Material umfasst, das eine andere Zusammensetzung hat als eine Zusammensetzung von einer der Schichten

21. Das Verfahren nach Anspruch 20, wobei das erste Material ein oder mehrere biokompatible Materialien beinhaltet, die geeignet sind Korngrenzen zwischen angrenzenden Schichten (25) zu unterbrechen.

22. Das Verfahren nach Anspruch 12, weiter umfassend Entfernen von Teilen des röhrenförmigen Teils um eine Mehrzahl von Bändern (22) und Verbindern (24) zu formen.

23. Das Verfahren nach Anspruch 22, wobei zumindest eines der Bänder (22) eine Dicke von 0,01 mm bis 0,08 mm hat.

24. Das Verfahren nach Anspruch 22, wobei das zumindest eine der Bänder (22) eine Mehrzahl von Schichten (25) umfasst.

25. Das Verfahren nach Anspruch 22, wobei der zumindest eine der Verbinder (24) eine Breite von 0,03 mm bis 0,3 mm hat.

## Revendications

1. Endoprothèse comportant au moins trois couches (25), chacune des couches comportant un ou plusieurs grains (29) dans une épaisseur de la couche (25), **caractérisée en ce que** les couches ont sensiblement la même composition, et **en ce qu'**une couche interfaciale (27) est disposée entre chacune des couches (25) pour empêcher les grains des couches adjacentes de fusionner les uns avec les autres, dans laquelle au moins une des couches comprend de l'acier inoxydable, un alliage comportant du platine et de l'acier inoxydable, du niobium, du tantale, du titane, de l'iridium, du cobalt, et/ou du chrome.

2. Endoprothèse selon la revendication 1, dans laquelle au moins certains des grains (29) ont un indice de grains selon la norme ASTM E1 12 d'au moins 6.

3. Endoprothèse selon la revendication 1, dans laquelle au moins certains des grains (29) ont un indice de grains selon la norme ASTM E1 12 de 6 à 12.

4. Endoprothèse selon la revendication 1, dans laquelle au moins une des couches (25) a une épaisseur de 0,08 mm ou moins.

5. Endoprothèse selon la revendication 4, dans laquelle l'épaisseur de ladite au moins une des couches (25) est de 0,01 mm à 0,08 mm

6. Endoprothèse selon la revendication 1, dans laquelle la couche interfaciale (27) comporte un premier matériau dont la composition est différente d'une composition des deux couches.

7. Endoprothèse selon la revendication 6, dans laquelle ledit premier matériau comprend un ou plusieurs matériaux biocompatibles pouvant interrompre les joints de grains entre les couches adjacentes (25).

8. Endoprothèse selon la revendication 6, dans laquelle le premier matériau comporte un alliage d'acier inoxydable.

9. Endoprothèse selon la revendication 1, comportant en outre une pluralité de bandes (22) et de connecteurs (24).

10. Endoprothèse selon la revendication 9, dans laquelle au moins une des bandes (22) comporte la pluralité de couches (25).

11. Endoprothèse selon la revendication 9, dans laquelle au moins une des bandes (22) a une épaisseur de 0,01 mm à 0,08 mm.

12. Procédé pour fabriquer une endoprothèse, le procédé consistant à :
former un organe tubulaire définissant une lumière dans l'endoprothèse, l'organe tubulaire comportant au moins trois couches (25), chacune des couches comportant un ou plusieurs grains (29) dans une épaisseur de la couche (25), dans lequel au moins une des couches comprend de l'acier inoxydable, un alliage comportant du platine et de l'acier inoxydable, du niobium, du tantale, du titane, de l'iridium, du cobalt, et/ou du chrome ;
**caractérisé en ce que** les couches (25) ont sensiblement la même composition, et **en ce que** le procédé consiste en outre à disposer une couche interfaciale (27) entre chacune des couches (25) pour empêcher les grains des couches adjacentes de fusionner les uns avec les autres.

13. Procédé selon la revendication 12, consistant en outre à faire diminuer l'épaisseur d'au moins une des couches (25).

14. Procédé selon la revendication 13, dans lequel après l'étape de diminution de l'épaisseur, l'épaisseur de ladite au moins une des couches (25) est de 0,08 mm ou moins.

15. Procédé selon la revendication 13, dans lequel après l'étape de diminution de l'épaisseur, l'épaisseur de ladite au moins une des couches (25) est de 0,01 mm à 0,08 mm

16. Procédé selon la revendication 12, consistant à disposer de manière concentrique une pluralité de tubes pour former l'organe tubulaire.

17. Procédé selon la revendication 12, dans lequel au moins certains des grains ont un indice de grains selon la norme ASTM E1 12 de 6 ou plus.

18. Procédé selon la revendication 17, dans lequel lesdits au moins certains des grains ont un indice de grains selon la norme ASTM E1 12 de 6 à 12.

19. Procédé selon la revendication 12, comportant en outre un travail consistant à durcir l'organe tubulaire de sorte que la taille d'au moins certains des grains diminue.

20. Procédé selon la revendication 12, dans lequel la couche interfaciale (27) comporte un premier matériau dont la composition est différente d'une composition de l'une des couches.

21. Procédé selon la revendication 20, dans lequel ledit premier matériau comprend un ou plusieurs matériaux biocompatibles pouvant interrompre les joints de grains entre les couches adjacentes (25).

22. Procédé selon la revendication 12, consistant en outre à enlever des parties de l'organe tubulaire pour former une pluralité de bandes (22) et de connecteurs (24).

23. Procédé selon la revendication 22, dans lequel au moins une des bandes (22) a une épaisseur de 0,01 mm à 0,08 mm.

24. Procédé selon la revendication 22, dans lequel ladite au moins une des bandes (22) comporte la pluralité de couches (25).

25. Procédé selon la revendication 22, dans lequel ledit au moins un des connecteurs (24) a une largeur de 0,03 mm à 0,3 mm.
